Europäisches Patentamt

European Patent Office

Office européen des brevets

(19)

(11) Veröffentlichungsnummer: **0 312 941**
**A2**

(12) ## EUROPÄISCHE PATENTANMELDUNG

(21) Anmeldenummer: 88117186.2

(22) Anmeldetag: 15.10.88

(51) Int. Cl.⁴: **C12N 9/72** , **C12N 15/00** , **A61K 37/02**

Die Bezeichnung der Erfindung wurde geändert (Richtlinien für die Prüfung im EPA, A-III, 7.3).

(30) Priorität: 23.10.87 DE 3735917

(43) Veröffentlichungstag der Anmeldung:
26.04.89 Patentblatt 89/17

(84) Benannte Vertragsstaaten:
AT BE CH DE ES FR GB IT LI NL

(71) Anmelder: **BASF Aktiengesellschaft**
**Carl-Bosch-Strasse 38**
**D-6700 Ludwigshafen(DE)**

(72) Erfinder: **Koerwer, Wolfgang, Dr.**
**Auf dem Leimen 10**
**D-6718 Grünstadt(DE)**
Erfinder: **Kurfürst, Manfred, Dr.**
**Anilinstrasse 71**
**D-6733 Hassloch(DE)**
Erfinder: **Baldinger, Verena, Dr.**
**Schiffsgasse 6**
**D-6900 Heidelberg(DE)**
Erfinder: **Doerper, Thomas, Dr.**
**Luitpoldstrasse 3**
**D-6719 Bissersheim(DE)**
Erfinder: **Schwarz, Margarete, Dr.**
**Leinhoehlweg 7**
**D-6705 Deidesheim(DE)**

(54) **Polypeptide mit Prourokinase-Aktivität, ihre Herstellung und Verwendung.**

(57) Es werden Polypeptide beschrieben, die sich von Prourokinase durch N-terminale Verkürzungen bzw. N-teminale Verkürzungen in Verbindung mit dem Austausch einer weiteren Aminosäure unterscheiden. Die Polypeptide werden gentechnisch hergestellt und besitzen thrombolytische Eigenschaften.

EP 0 312 941 A2

EP 0 312 941 A2

## Neue Polypeptide, ihre Herstellung und Verwendung

Die vorliegende Erfindung betrifft neue Polypeptide mit Prourokinase-Aktivität, Verfahren zu deren Herstellung und deren Verwendung bei der Bekämpfung von Krankheiten.

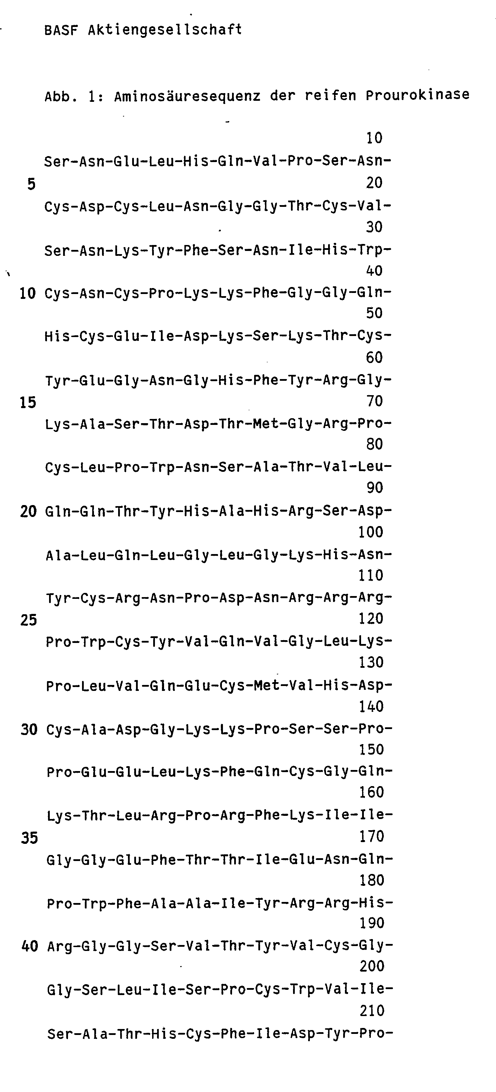

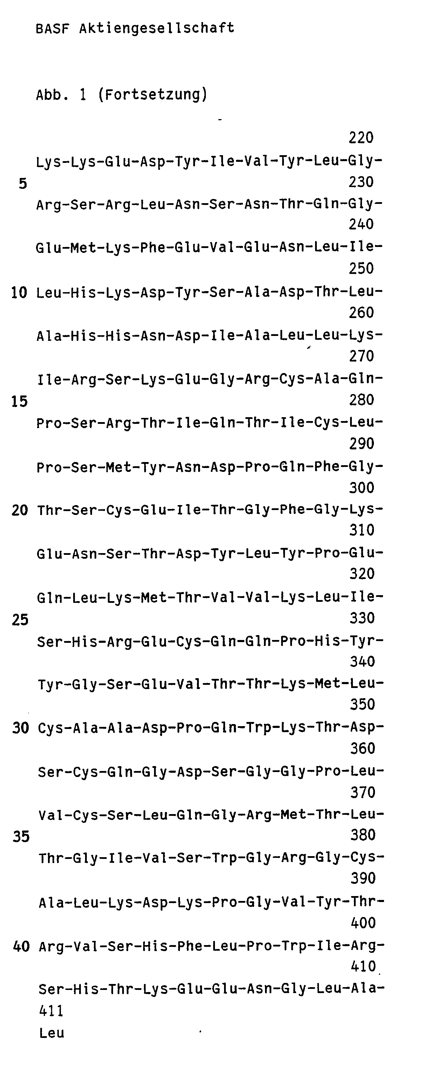

Der Plasminogenaktivator Prourokinase (PUK), auch "single chain urokinase plasminogenactivator (SCUPA)" oder "kidney plasminogenactivator" (KPA) genannt, ist ein Einketten-Polypeptid aus 411 Aminosäuren und einem Molekulargewicht von etwa 54 kD. Abb. 1 zeigt die Aminosäuresequenz der reifen Prourokinase (Z. Physiol. Chem. 363, 133, 1043, 1155 (1982). Aus der Prourokinase entsteht durch limitierte Proteasespaltung der Lys$^{157}$-Phe$^{158}$-Bindung ein Zweikettenmolekül, die hochmolekulare Urokinase (HUK). Diese besitzt dasselbe Molekulargewicht wie die Prourokinase. Durch eine weitere proteolytische Spaltung zwischen Lys$^{135}$ und Lys$^{136}$ entsteht die niedermolekulare Urokinase (LUK) mit einem Molekulargewicht von etwa 33 kD. Die LUK ist wie die HUK ein Zweikettenmolekül. Alle drei Formen sind im humanen Urin oder im humanen Serum als natürliche Formen nachgewiesen worden (J. Biol. Chem. 257, 3276 (1982) und 261, 1267 (1986)). Eine weitere Form wurde aus Überständen der humanen Tumorzellinie CALU-3 (ATCC, HTB-55) isoliert, 32 kD scuPA. Diese Prourokinaseform ist ein Einkettenmolekül, bestehend aus den Aminosäuren 144-411 (J. Biol. Chem. 261, 17120-17126 (1986).

Sowohl die Ein-, als auch die Zweikettenformen besitzen fibrinolytische Eigenschaften. Die Zweikettenmoleküle wirken allerdings systemisch. Sie degradieren sowohl Fibrin als auch Fibrinogen überall im Körper und es kommt bei der Applikation häufig zu lebensbedrohenden Blutungen. Allein die Einkettenformen sind gerinnselspezifisch und zeigen nur eine geringe systemische Lyse.

Es wurde nun gefunden, daß Polypeptide der Formel I

R-PUK$^{144-155}$-X-PUK$^{157-411}$

worin R

$$Y-Lys-Pro-Ser-Ser-Pro-Pro-Glu-Glu,$$
$$Y-Pro-Ser-Ser-Pro-Pro-Glu-Glu,$$
$$Y-Ser-Ser-Pro-Pro-Glu-Glu,$$
$$Y-Ser-Pro-Pro-Glu-Glu,$$
$$Y-Ser-Pro-Glu-Glu,$$
$$Y-Pro-Glu-Glu,$$
$$Y-Glu-Glu,$$
$$Y-Glu \text{ oder}$$
$$Y$$

(mit Y in der Bedeutung von Wasserstoff, Ala oder Met), PUK$^{144-155}$ und PUK$^{157-411}$ für die Aminosäuren 144 bis 155 und 157 bis 411 der Prourokinase und X eine der üblichen L-$\alpha$-Aminosäuren darstellen, wobei jedoch X nicht Arg sein darf, wenn Y Wasserstoff ist, verbesserte Eigenschaften besitzen, wie beispielsweise eine verlängerte Serumhalbwertzeit und vergrößerte enzymatische Stabilität.

Als übliche Aminosäuren sind Ala, Arg, Asn, Asp, Cys, Gln, Glu, Gly, His, Ile, Leu, Lys, Met, Phe, Pro, Ser, Thr, Trp, Try und Val zu verstehen.

Die Polypeptide der Formel I können an der Aminosäure 302 (Asn, vgl. Abb. 1) einen Glykosylrest tragen.

Die Aminosäurereste 144 bis 155 und 157 bis 411 der Prourokinase sind aus Abb. 1 ersichtlich.

Die Erfindung betrifft weiter DNA-Sequenzen, die für die Polypeptide der Formel I kodieren, sowie Vektoren, die diese DNA-Sequenzen enthalten.

Die neuen Polypeptide lassen sich gentechnologisch nach bekannten Verfahren herstellen. Ausgangspunkt für die hier beschriebenen Konstruktionen ist ein cDNA-Klon, im folgenden pU20 genannt, der die kodierende Sequenz von Prourokinase und noch 5'- und 3'- nicht translatierte Bereiche beinhaltet. Die Teilsequenz des cDNA-Klons, der die kodierende Region umfaßt und aus der sich die Proteinsequenz (Abb.

2

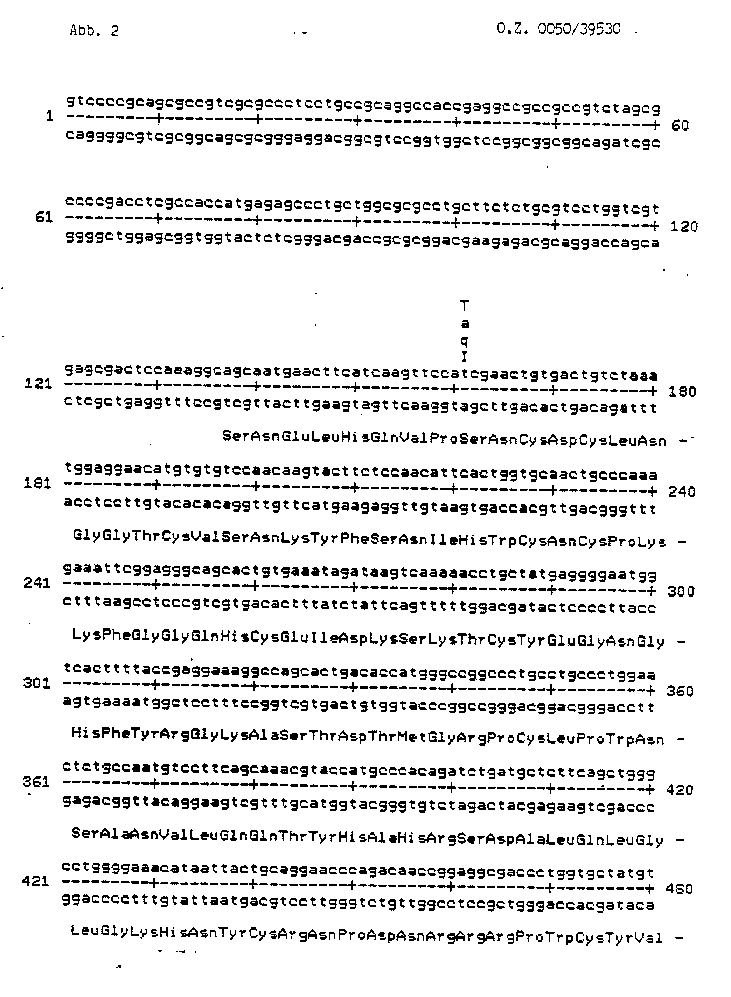

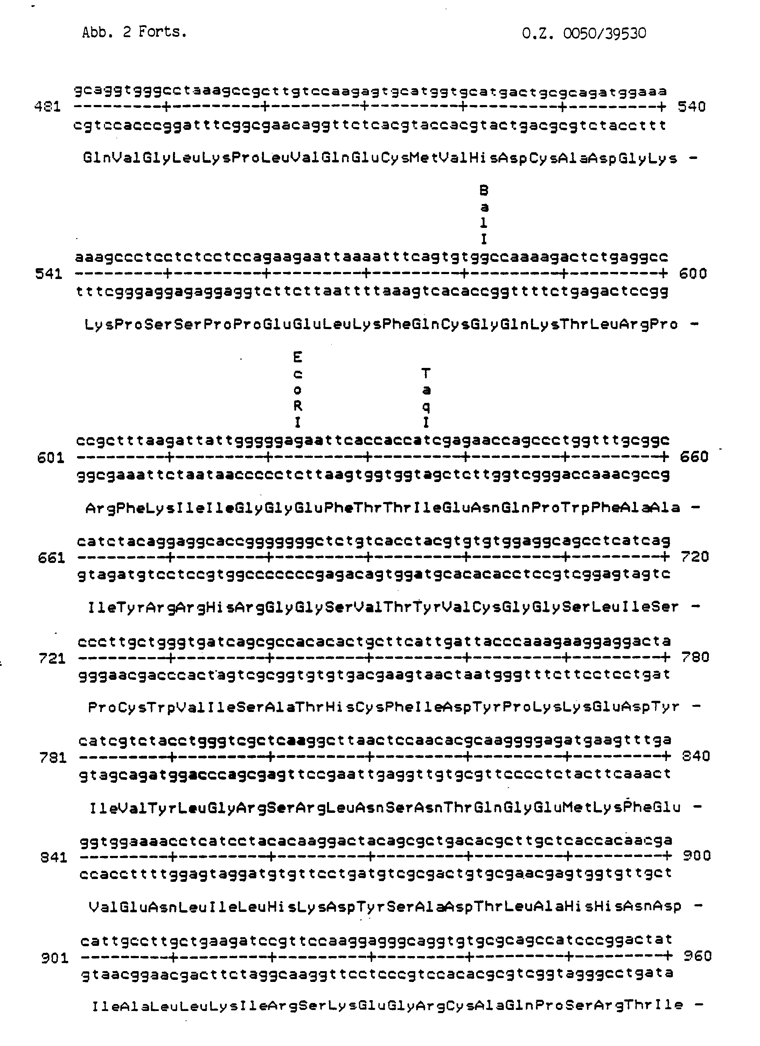

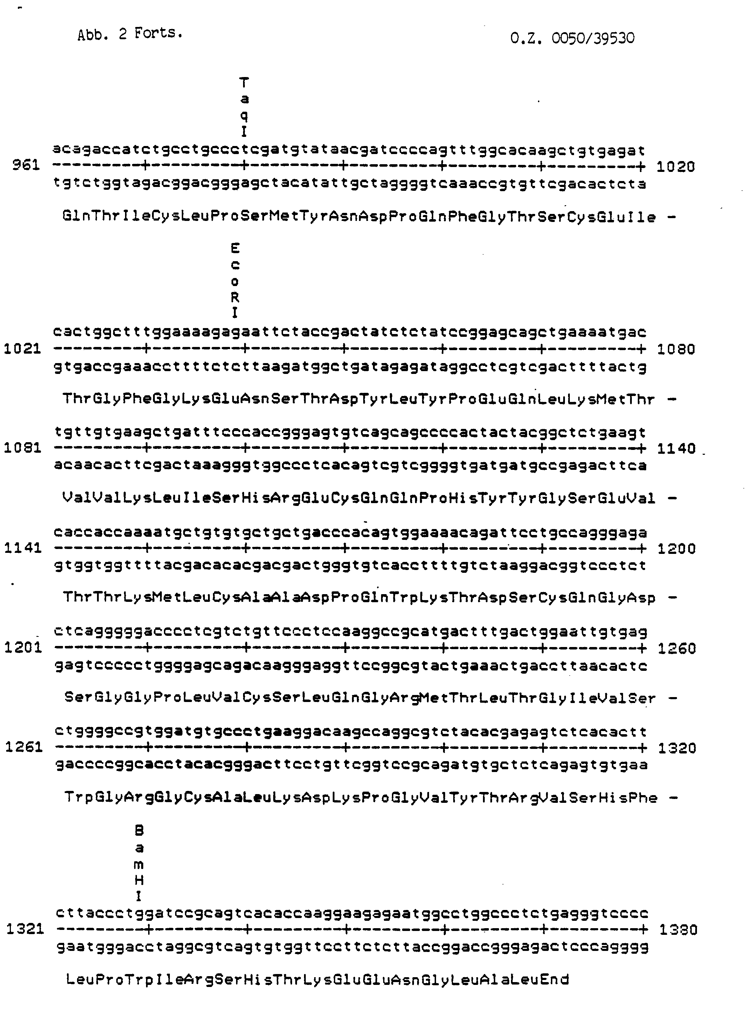

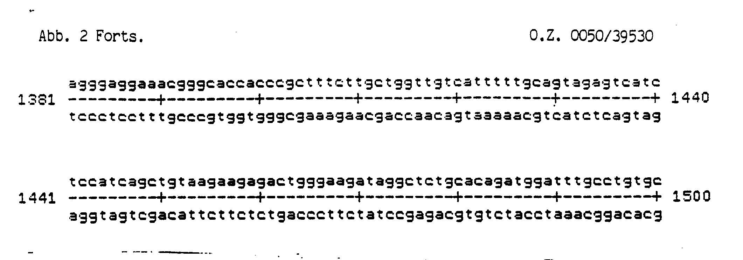

1) ableitet, ist in Abb. 2 dargestellt. Das reife Protein beginnt nach einer sogenannten "leader"-Sequenz bei Ser[1] und terminiert nach Leu[+11].

pU20 wurde aus der humanen, Prourokinase produzierenden Tumorzellinie Detroit 562 (ATCC No. CCL 138) erhalten: Aus dieser Zellinie wurde m-RNA isoliert und in doppelsträngige cDNA umgeschrieben.

Nach Einsetzen dieser cDNA in den kommerziell erhältlichen Klonierungsvektor puc 18 wurde eine cDNA-Bibliothek angelegt. Die dabei verwendeten Methoden sind beispielsweise in Maniatis et al, "Molecular Cloning", CSH-Press nachzulesen. Auch das "Screenen" solcher Genbänke mit radioaktiv markierten Oligonukleotidsonden ist inzwischen eine vielfach verwendete und beschriebene Methode.

Teile der DNA-Sequenz von pU20 sind mit Hilfe von Restriktionsendonukleasen leicht zugänglich. Diese Fragmente, gegebenenfalls in Verbindung mit chemisch synthetisierten Oligonukleotiden, Adaptoren oder Genfragmenten können benutzt werden, um die DNA-Sequenzen, die für die neuen Polypeptide kodieren, zu klonieren. Der Einbau der Genfragmente bzw. synthetischen DNA-Sequenzen in Klonierungsvektoren, beispielsweise die handelsüblichen Plasmide pBR 322, puc 18 und puc 19, erfolgt in bekannter Weise. Auch können die Gene oder Genfragmente mit geeigneten chemisch synthetisierten oder aus Bakterien bzw. Phagen isolierten Kontrollregionen versehen werden, die die Expression der Proteine ermöglichen. Die Transformation der so erhaltenen Hybridplasmide in geeignete Wirtsorganismen ist ebenfalls bekannt und eingehend beschrieben. Auch können die Hybridplasmide mit entsprechenden Signalsequenzen versehen werden, die die Sekretion der Polypeptide in das Periplasma von E.coli erlauben.

Eine Expression in Mammalia-Zellkultur bzw. Hefe oder Insektenzellen ist in analoger Weise möglich: Hierbei müssen allerdings eukaryontische Kontrollregionen verwendet werden. Bei der Expression in Mammaliazellen kann man Vektoren verwenden, die z.B. das zu exprimierende Fremdgen unter die Kontrolle des viralen SV 40 Promotors setzt. Solche Plasmide besitzen keinen eukaryontischen Replikationsursprung. Man isoliert nach der Transformation in permissive Mammaliazellen Klone, die Kopien dieser Plasmide integriert im Genom tragen. In gleicher Weise kann man die zu exprimierenden Gene unter der Kontrolle eines eukaryontischen Promotors auf Vektoren auf der Basis des Rinderpapillom-Virus klonieren. Solche Expressionsplasmide bleiben episomal in einer Kopienzahl von 20 bis 100 in der permissiven Mausfibroblastenzellinie C127.

Diese eukaryontischen Expressionssysteme besitzen den Vorteil, daß sie in der Lage sind, ihre Produkte effektiv und meist in nativer Form zu sekretieren. Ferner besitzen sie die Fähigkeit, ihre Produkte posttranslationell zu modifizieren.

So erhält die Prourokinase bei einer Expression in Eukaryontenzellen noch eine Glykosidseitenkette an der Aminosäure 302 (Asn). Bakterien sind nicht in der Lage, Glycosidseitenketten zu synthetisieren. Die meisten in Bakterien exprimierten eukaryontischen Proteine, wie auch die Prourokinase und die von ihr erfindungsgemäß abgeleiteten Polypeptide, fallen in der Zelle als denaturierte Einschlußkörper an und müssen proteinchemisch zurückgefaltet werden. Ferner sind Bakterien oft nicht in der Lage, die Initiatoraminosäure Methionin vom fertigen Protein abzuspalten. Die Abspaltung des endständigen Methionins bei der Expression in Bakterien und in manchen Fällen die Verhinderung der Bildung von Einschlußkörpern kann durch die Verwendung von Sekretionssystemen erreicht werden. Bei der Verwendung solcher Systeme besitzt dann allerdings häufig der N-Terminus noch eine für die Spaltstelle wichtige Aminosäure, wie z.B. Alanin. Obwohl die Expression in Bakterien viele Probleme aufwirft, sind diese, wie bei der Prourokinase das Expressionssystem der Wahl, wenn sich das anfallende Rohprotein zum aktiven Enzym renaturieren läßt. Daher wird hier die Expression in Bakterien bevorzugt.

Aufgrund der Degeneration des genetischen Codes ist es auch möglich, andere DNA-Sequenzen, z.B. chemisch synthetisierte Prourokinasegene oder Teile davon mit anderen Tripletts als die hier beschriebenen für die Expression der neuen Polypeptide zu benutzen.

Die neuen Wirkstoffe können zur Thrombolyse bei arteriellen und venösen Verschlußkrankheiten eingesetzt werden und zeigen verbesserte Eigenschaften gegenüber humaner Prourokinase.

Gegenstand der Erfindung sind daher auch Arzneimittel, die mindestens eines der neuen Polypeptide enthalten, gegebenenfalls in einem pharmazeutisch verträglichen Träger oder Bindemittel.

Die neuen Proteine können auch zusammen mit anderen Fibrinolytika, wie Urokinase, TPA, Streptokinase, von diesen drei Proteinen abgeleiteten Derivaten oder Natriumpentosanpolysulfat, angewendet werden.

Weitere Ausgestaltungen der Erfindung sind in den folgenden Beispielen näher beschrieben.

Beispiele

1. Isolierung eines cDNA-Klons für humane Prourokinase

Die Prourokinase produzierende humane Tumorzellinie Detroit 562 (ATCC Nr. CCL 138) wurde in "Eagle's minimal essential"-Medium mit 3 % NaHCO₃, 10 % fötalem Kälberserum, 1 % Aminosäuren, 2,4 % Hepes pH 7,5 bei 37°C und 5 % CO₂-Atmosphäre bis zur Konfluenz gezogen. Das Medium wurde abgegossen, die Zellen mit physiologischer Kochsalzlösung gewaschen und in Lysepuffer (6M Guanidiniumisothiocyanat, 5 mM Natriumcitrat (pH 7) 0,1 M 2-Mercaptoethanol, 0,5 % Sarkosyl) aufgeschlossen. Die RNA wurde durch ein 5,7 M CsCl-Kissen über Nacht bei 100.000 g sedimentiert. Die polyA+-RNA enthaltende Fraktion wurde durch zweimalige Affinitätschromatographie an oligo(dT)-Cellulose abgetrennt.

Mit Hilfe des Enzyms AMV-Reverse Transkriptase und oligo(dT)12-18 als Starter wurde die polyA+-RNA in einsträngige cDNA umgeschrieben. Die Synthese des zweiten Strangs erfolgte mit E.coli-DNA-Polymerase I. An die doppelsträngige cDNA wurde mit Hilfe des Enzyms Terminale Transferase an jedem 5'-Ende ca. 10-20 dG-Reste ansynthetisiert. Ebenso wurde mit dem kommerziell erhältlichen Plasmid puC 18 verfahren, das mit der Restriktionsendonuklease SphI linearisiert wurde und an dessen 5'-Enden wie oben beschrieben ca. 10 bis 20 dC-Reste ansynthetisiert wurden. Beide DNAs wurden miteinander verschmolzen und das Hybrid in kompetente Zellen des E.coli-Stammes HB 101 transformiert. Die Klone wurden auf LB-Platten mit 100 μg/ml Ampicillin als Selektionsmarker ausplattiert und die Kolonien auf Nitrocellulose übertragen.

Die Bakterien auf den Filtern wurden repliziert, lysiert und die denaturierte DNA fest an das Filter gebunden.

Mit Hilfe eines DNA-Synthesizers (Applied Biosystems, Typ 380A) wurden vier 17er Oligonukleotidsonden mit Homologie zur publizierten DNA-Sequenz der humanen Prourokinase hergestellt. Diese Sonden hatten folgede Sequenzen:

5' TCAGCAGCACATAGCAT 3'
5' AACCAGGGCTGGTTCTC 3'
5' ACCATGCACTCTTGGAC 3'
5' TGCTGGTCACAGGTCAT 3'

Die Oligonukleotidsonden wurden am 5'-Ende mit γ-³²P-ATP markiert und die Nitrocellulosefilter in 6xSET Puffer (1xSET = 0,15 M NaCl, 0,015 M Tris-HCl (pH 7,4) 0,001 M EDTA) 0,1 % SDS und 10 % Dextransulfat über Nacht bei 42°C unter Schütteln mit den Sonden inkubiert. Nach intensivem Waschen der Filter in 6 x SET/0,1 % SDS bei 42°C wurden die Filter mit einem Röntgenfilm exponiert und Klone mit Sequenzhomologie ermittelt. Die homologen Klone wurden isoliert und vereinzelt.

Einer der so isolierten Klone war pU 20. Abb. 2 zeigt die Sequenz der kodierenden Region und benachbarter Bereiche.

2. Herstellung eines Hybridplasmids für die Expression der Vergleichssubstanz Prourokinase

Ausgangspunkt war das Plasmid pU20 (Beispiel 1). Aus diesem wurde durch eine limitierte TaqI/BamHI-Verdauung das Fragment des Prourokinasegens von Nukleotid 162 bis 1329 herausgeschnitten und gelelektrophoretisch isoliert (Fragment 1). Das Plasmid puc 18 wurde mit EcoRI und SalI linarisiert, dadurch entstand das Fragment 2.

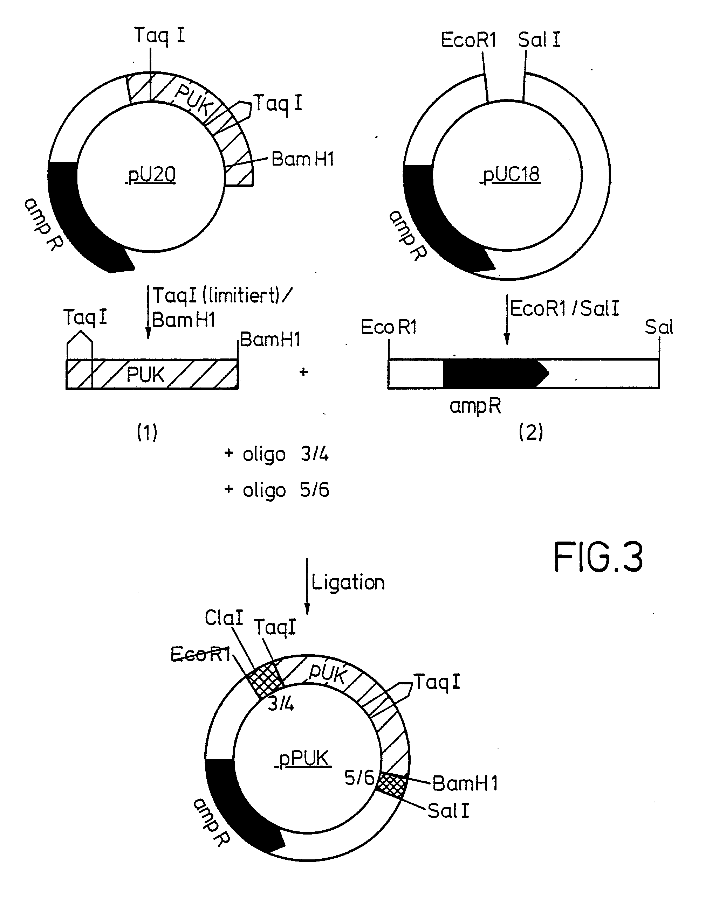

Fragmente 1 und 2 wurden mit den Oligonukleotidadaptoren 3/4 und 5/6 ligiert, die das jeweilige 5'-Ende (3/4) bzw. 3'-Ende (5/6) des Prourokinasegens wieder herstellen. Durch die Verwendung von Oligonukleotid 3/4 wurde die EcoRI-Schnittstelle am 5'-Ende deletiert. So erhielt man gemäß Abb. 3 das Hybridplasmid pPUK, das das Prourokinase-Gen als leicht klonierbare ClaI/SalI-"Kassette" zum Umsetzen in unterschiedliche Expressionsvektoren verfügbar machte.

```
        EcoRI    ClaI                                                    TaqI
                          Met Ser Asn Glu Leu His Gln Val Pro
    3/4 5' |AATTGA|CGAT ATG AGC AAT GAA CTT CAT CAA GTT CCA T|___        3'
        3'       |CTAGC|TA TAC TCG TTA CTT GAA GTA GTT CAA GGT AGC|      5'


        BamHI
                  Ile Arg Ser His Thr Lys Glu Glu Asn Gly Leu
    5/6 5' |G ATC CGC AGT CAC ACC AAG GAA GAG AAT GGC CTG
        3'       |GCG TCA GTG TGG TTC CTT CTC TTA CCG GAC


                        SalI
        Ala Leu Stop |
        GCC CTC TGA G|___           3'
        CGG GAG ACT CAGCT|          5'
```

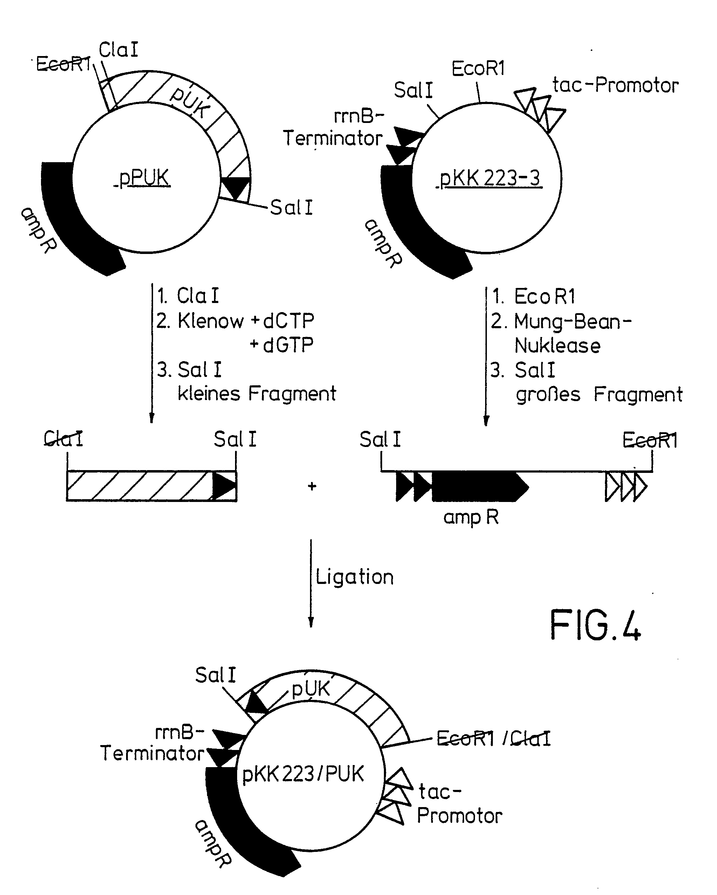

Das Hybridplasmid pPUK wurde mit ClaI geöffnet und die überstehenden 5'-Enden mit Klenow Fragment der DNA Polymerase I und dCTP und dGTP aufgefüllt. Das Fragment mit dem Urokinasegen erhielt man durch Nachschneiden mit der Endonuklease SalI und gelelektrophoretischer Aufreinigung. Dieses Fragment wurde in den kommerziell erhältlichen Expressionsvektor pKK 223-3 (Pharmacia Bestell-Nr. 27-4935-01) eingesetzt, den man mit EcoRI geöffnet, die überhängenden Enden mit "mung bean-nuclease" (mung bean = Mungbohne) nach Angaben des Herstellers entfernt und anschließend mit SalI nachgeschnitten hatte. Nach der Ligation der Fragmente erhielt man das Expressionsplasmid pKK 223/PUK gemäß Abb. 4. Das Plasmid wurde in kompetent gemachte Zellen des E.coli Stammes K12 JM105 (Pharmacia Bestell-Nr. 27-1550-01) transformiert. Dieser Stamm enthält das Episom F'iᵠ mit dem lac-Repressor, der unter den üblichen Wachstumsbedingungen das Prourokinasegen reprimiert. Durch Einstellen des Gehalts an Isopropyl-$\beta$-D-thiogalactosid (IPTG) auf 0,5 mM in der logarithmischen Wachstumsphase wurde die Expression des Prourokinasegens induziert. Dabei fiel die Prourokinase intrazellulär denaturiert als sogenannte "Einschlußkörper" an. Diese konnten nach Aufschluß der Bakterien gereinigt und daraus die Prourokinase proteinchemisch renatuiert und aufgereinigt werden.

3. Herstellung der Hybridplasmide für die Expression der N-terminalen Verkürzungen

Das Plasmid pPUK (Abb. 3, Beispiel 2) wurde über den Methylase-negativen Stamm GM 1813 passagiert. Anschließend wurde mit einer ClaI/BalI-Spaltung das 5'-Ende des Prourokinasegens bis einschließlich Nukleotid 582 entfernt. Das 5'-Ende wurde mit Hilfe synthetischer Oligonukleotide ergänzt und es entstanden die Gensequenzen, die für die neuen Polypeptide kodieren. Durch die Ligation mit den synthetischen DNA-Fragmenten Δ135 bis Δ143:

Δ135:

```
   ClaI                                                                          BalI
          Met Lys Pro Ser Ser Pro Pro Glu Glu Leu Lys Phe Gln Lys
   5' CGAT ATG AAG CCC TCC TCT CCT CCA GAA GAA TTA AAA TTT CAG TGT GG
   3'  TA TAC TTC GGG AGG AGA GGA GGT CTT CTT AAT TTT AAA GTC ACA CC
```

Δ136:

```
   ClaI                                                                       BalI
          Met Pro Ser Ser Pro Pro Glu Glu Leu Lys Phe Gln Lys
   5' CGAT ATG CCC TCC TCT CCT CCA GAA GAA TTA AAA TTT CAG TGT GG
   3'  TA TAC GGG AGG AGA GGA GGT CTT CTT AAT TTT AAA GTC ACA CC
```

Δ137:

```
   ClaI                                                                    BalI
          Met Ser Ser Pro Pro Glu Glu Leu Lys Phe Gln Lys
   5' CGAT ATG TCC TCT CCT CCA GAA GAA TTA AAA TTT CAG TGT GG
   3'  TA TAC AGG AGA GGA GGT CTT CTT AAT TTT AAA GTC ACA CC
```

Δ138:

```
ClaI                                                                    BalI
        |        Met Ser Pro Pro Glu Glu Leu Lys Phe Gln Lys            |
     5' |CGAT ATG TCT CCT CCA GAA GAA TTA AAA TTT CAG TGT GG|
     3'  |TA TAC AGA GGA GGT CTT CTT AAT TTT AAA GTC ACA CC|
```

Δ139:

```
ClaI                                                            BalI
        |        Met Pro Pro Glu Glu Leu Lys Phe Gln Lys        |
     5' |CGAT ATG CCT CCA GAA GAA TTA AAA TTT CAG TGT GG|
     3'  |TA TAC GGA GGT CTT CTT AAT TTT AAA GTC ACA CC|
```

Δ140:

```
ClaI                                                        BalI
        |        Met Pro Glu Glu Leu Lys Phe Gln Lys         |
     5' |CGAT ATG CCA GAA GAA TTA AAA TTT CAG TGT GG|
     3'  |TA TAC GGT CTT CTT AAT TTT AAA GTC ACA CC|
```

Δ141:

```
ClaI                                                    BalI
        |        Met Glu Glu Leu Lys Phe Gln Lys         |
     5' |CGAT ATG GAA GAA TTA AAA TTT CAG TGT GG|
    .3'  |TA TAC CTT CTT AAT TTT AAA GTC ACA CC|
```

Δ142:

```
ClaI                                                BalI
        |        Met Glu Leu Lys Phe Gln Lys         |
     5' |CGAT ATG GAA TTA AAA TTT CAG TGT GG|
     3'  |TA TAC CTT AAT TTT AAA GTC ACA CC|
```

Δ143:

```
ClaI                                            BalI
        |        Met Leu Lys Phe Gln Lys         |
     5' |CGAT ATG TTA AAA TTT CAG TGT GG|
     3'  |TA TAC AAT TTT AAA GTC ACA CC|
```

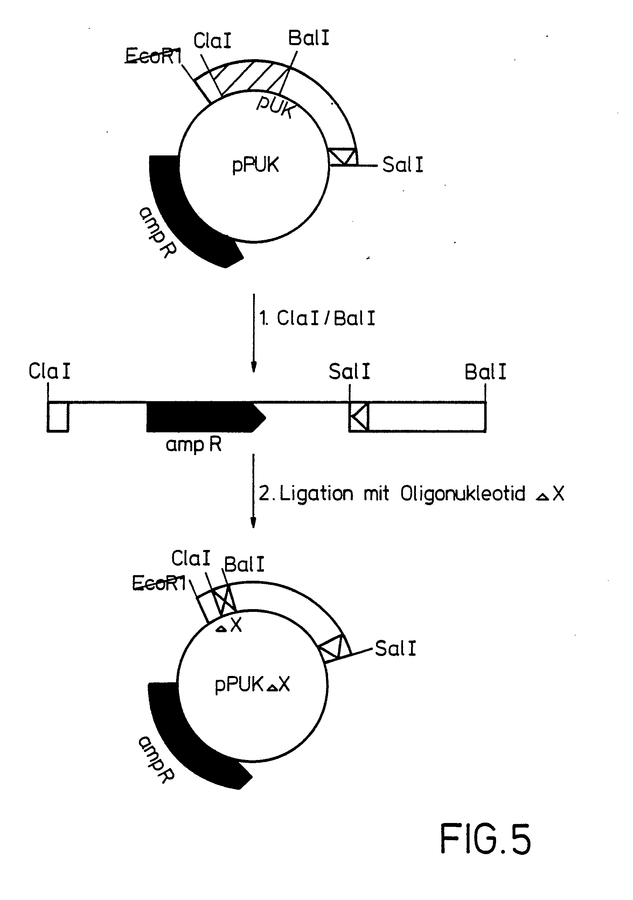

entstanden die Hybridplasmide pPUKΔ135 bis pPUKΔ143 im folgenden vereinfacht als pPUKΔX bezeichnet (Abb. 5). Mit Hilfe dieser DNA-Sequenzen wurden wie in Beispiel 2 beschrieben die erfindungsgemäß beanspruchten Polypeptide gentechnologisch in E.coli synthetisiert.

4. Herstellung der Hybridplasmide für die Expression von verkürzter PUK mit Aminosäureaustausch in Position 156

Die Hybridplasmide pPUKΔX (siehe Beispiel 3, Abb. 5) wurden mit BalI vollständig und mit EcoRI partiell gespalten. Das den Vektor enthaltende Fragment wurde gelelektrophoretisch aufgereinigt und mit den synthetischen Oligonukleotiden 84/85, 86/87 oder 88/89 ligiert:

7

```
        156    156
      Arg——→Lys                                    156
            BalI Gln Lys Thr Leu Arg Pro Lys Phe Lys Ile Ile
         5' |C CAA AAG ACT CTG CGT CCC AAG TTT AAG ATT ATT
   84/85 3' |G GGT TTC TGA GAC GCA GGG GTC AAA TTC TAA TAA


                                   Pro Pro          EcoRI
                                   GGC GGT G|‾‾‾‾‾‾‾‾
                                   CCG CCA C TTAA|


        156    156
      Arg——→Gln                                    156
            BalI Gln Lys Thr Leu Arg Pro Gln Phe Lys Ile Ile
         5' |C CAA AAG ACT CTG CGT CCC CAG TTT AAG ATT ATT
   86/87 3' |G GTT TTC TGA GAC GCA GGG GTC AAA TTC TAA TAA


                                   Pro Pro          EcoRI
                                   GGC GGT G|‾‾‾‾‾‾‾‾
                                   CCG CCA C TTAA|


        156    156
      Arg——→Leu                                    156
            BalI Gln Lys Thr Leu Arg Pro Leu Phe Lys Ile Ile
         5' |C CAA AAG ACT CTG CGT CCC CTC TTT AAG ATT ATT
   84/85 3' |G GTT TTC TGA GAC GCA GGG GAG AAA TTC TAA TAA


                                   Pro Pro          EcoRI
                                   GGC GGT G|‾‾‾‾‾‾‾‾
                                   CCG CCA C TTAA|
```

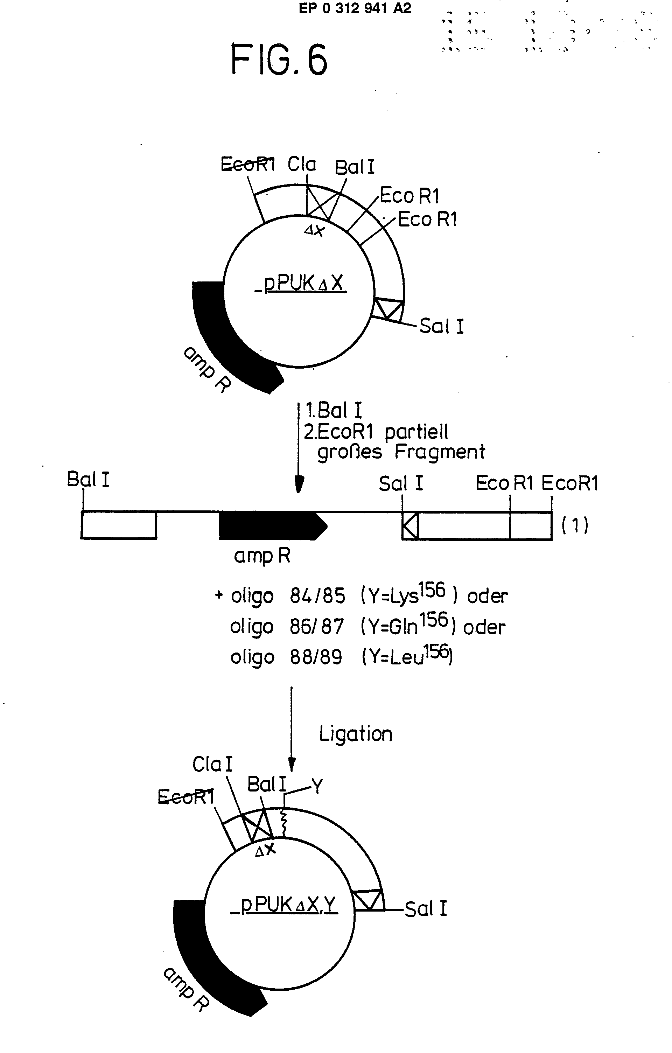

So enstanden die Hybridplasmide pPUKΔXY mit Y in der Bedeutung von Lys[156], Gln[156], Leu[156] (Abb. 6). Mit Hilfe dieser DNA-Sequenzen wurden wie in Beispiel 2 beschrieben die erfindungsgemäß beanspruchten Polypeptide gentechnologisch in E.coli hergestellt.

5. Aufreinigung und Renaturierung der in E.coli exprimierten Polypeptide

Die oben beschriebenen Polypeptide der Prourokinase lassen sich nach ihrer Expression in E.coli nach einem Standardverfahren aufreinigen und renaturieren:

Das Zellpellet (100 g Naßgemisch) wird in 500 ml (50 mM Phosphatpuffer pH 8,0, 10 mM EDTA, 1 mM DTT und 0,005 % Tween 80) aufgenommen und resuspendiert. Die mittels Ultraschall aufgeschlossenen Zellen werden abzentrifugiert und das Pellet mit den Einschlußkörpern mit 50 mM Phosphatpuffer, pH 8,0, 1 M NaCl, 1 mM DTT, 0,005 % Tween 80 gewaschen.

Die gewaschenen Einschlußkörper werden abzentrifugiert (10 min, 10.000 g) und in 6 M Guanidiniumhydrochlorid gelöst. Dies geschieht durch Rühren für 30 min bei Raumtemperatur in 200 ml 50 mM Phosphatpuffer pH 8,0, 5 mM DTT, 1 mM EDTA, 6 M Guanidiniumhydrochlorid und 0,005 % Tween 80. Nicht gelöstes Protein wird abzentrifugiert. Die Renaturierung des PUK-Muteins geschieht durch Ausverdünnung dieser Lösung auf eine Proteinkonzentration zwischen 0,2 und 0,3 mg/ml in 50 mM Phosphatpuffer pH 9,0, 1,5 M Guanidiniumhydrochlorid, 0,5 M Arginin, 1 mM GSH, 0,1 mM GSSG, 1 mM EDTA, 0,005 % Tween 80.

Die Proteinreinigung erfolgt durch eine Ammoniumsulfatpräzipitation, Affinitätschromatographie und Ionenaustauschchromatographie. Dazu wird die Renaturierungslösung mit Ammoniumsulfat (zwischen 40 bis 70 %) versetzt, das Pellet in 50 mM Phosphat, 1 M Guanidiniumhydrochlorid, 0,1 M NaCl, 0,005 % Tween 80 aufgenommen und auf eine Zn-Chelat-(Pharmacia)-Chromatographiesäule aufgetragen und mit 50 mM EDTA in obigem Puffer bei 4°C eluiert. Das eluierte Protein wird bei 4°C gegen 10 mM Phosphat, 10 mM NaCl, 0,005 % Tween 80 dialysiert und auf eine S-Sepharose (Pharmacia) aufgetragen. Das PUK-Mutein wird mit 50 mM Phosphat, 400 mM NaCl, 0,005 % Tween 80 von der Säule eluiert.

Die spezifischen Aktivitäten der gereinigten Proteine im direkten amidolytischen Test mit dem Substrat S-2444 (Kali-Vitrum) liegen im Bereich von 0,5 bis $1,5 \times 10^5$ U/mg.

6. Verstärkte Proteaseresistenz der PUK-Polypeptide mit Aminosäureaustausch in Position 156

Durch eine Thrombinspaltung der Prourokinase hinter Arg 156 entsteht ein 2-Ketten-Molekül, das nicht mehr aktivierbar ist und für eine Gerinnsel-Lyse nicht mehr zur Verfügung steht. Die oben beschriebenen Polypeptide mit Aminosäureaustausch in Position 156 sind nicht mehr oder in signifikant geringerem Ausmaß durch Thrombin inaktivierbar.

Stellvertretend für die oben beschriebenen Polypeptide soll diese verbesserte Eigenschaft für jene Moleküle demonstriert werden, die in Position 156 ein Glutamin oder ein Leucin tragen:

Der Thrombininaktivierungstest wurde wie folgt durchgeführt. Prourokinase (bzw. deren Muteine) wird auf 6000 U/ml verdünnt (50 mM Phosphat, pH 6,7, 300 mM NaCl, 0,005 % Tween 80) und mit Thrombin (5 U/ml) bei 37°C inkubiert. In Zeitabständen von 15 min werden Aliquote entnommen und im amidolytischen Test gemessen.

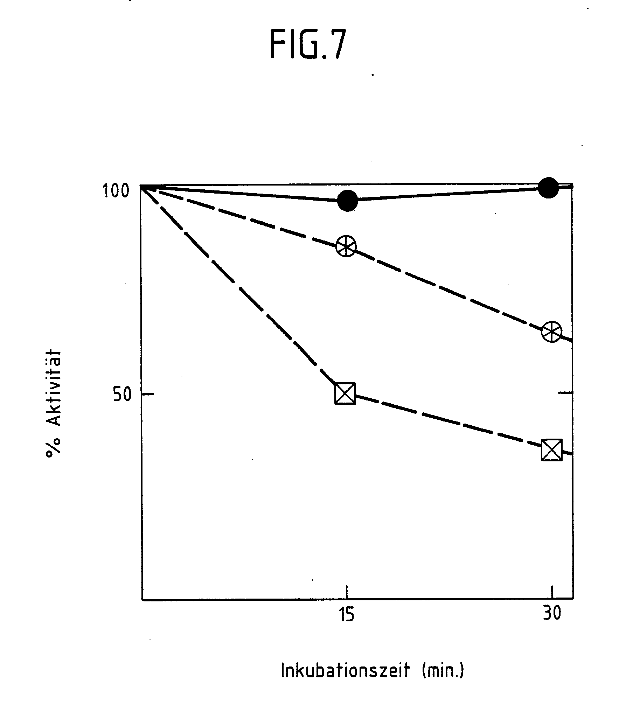

Es zeigte sich (vgl. Abb. 7), daß die Aktivität der Prourokinase im amidolytischen Test mit steigenden Thrombineinheiten abnimmt. So hat die aktivierte Prourokinase mit 2 U (∗ --x) Thrombin nach Inkubation von 15 min noch 86 %, nach 30 min noch 59 % seiner Ausgangsaktivität. Während mit 10 U Thrombin bei gleicher Inkubationsdauer (x...x) noch 50 % bzw. 36 % der Anfangsaktivität gemessen werden konnte. Eine Inkubation der Prourokinase unter identischen Bedingungen ohne Thrombin zeigte über einen Zeitraum von 1 h immer noch 100 % der Ausgangsaktivität. An Stelle der Prourokinase wurde nun das PUK-Mutein (Gln 156 oder Leu 156) im Thrombininhibierungstest eingesetzt. Abb. 7 macht deutlich, daß dieses Mutein nach 30 min Inkubation mit 5 U Thrombin noch 100 % der Ausgangsaktivität zeigt (o-o). Der Aminosäureaustausch an der Stelle 156 der Prourokinase (Arg→Gln) oder Leu bewirkt demnach, daß Thrombin am Mutein nicht mehr proteolytisch wirksam ist und kein inaktives Zweikettenmaterial entsteht.

7. Verlängerte in vivo Halbwertszeit

Die in Beispiel 3 bis 5 beschriebenen Polypeptide besitzen eine signifikant erhöhte in vivo Halbwertszeit. Stellvertretend soll dies am Beispiel der Polypeptide der Formel I beschrieben werden, in denen Y = Met und X = Arg (A) bzw. Y = Met und X = Gln (B) ist.

Pharmakokinetische Untersuchungen obengenannter Verbindungen an männlichen Sprague-Dawley Ratten von ca. 350 g zeigten nach i.v. Bolusinjektion von 1 mg/kg eine im Vergleich zu PUK verlängerte Halbwertszeit.

Hierfür wurden die Plasmaaktivitäten der Verbindungen nach Aktivierung mit Plasmin im amidolytischen Test mit dem chromogenen Substrat S 2444 (Kali-Vitrum) bestimmt:

| Verbindung | Halbwertszeit (min) |
|---|---|
| PUK | 3 |
| A | 10 |
| B | ≧10 |

**Ansprüche**

1. Polypeptide der Formel I

R-PUK$^{144-155}$-X-PUK$^{157-411}$

worin R

$$Y-Lys-Pro-Ser-Ser-Pro-Pro-Glu-Glu,$$
$$Y-Pro-Ser-Ser-Pro-Pro-Glu-Glu,$$
$$Y-Ser-Ser-Pro-Pro-Glu-Glu,$$
$$Y-Ser-Pro-Pro-Glu-Glu,$$
$$Y-Ser-Pro-Glu-Glu,$$
$$Y-Pro-Glu-Glu,$$
$$Y-Glu-Glu,$$
$$Y-Glu \text{ oder}$$
$$Y$$

(mit Y in der Bedeutung von Wasserstoff, Ala oder Met),
PUK$^{144-155}$ und PUK$^{157-411}$ die Aminosäuren 144 bis 155 und 157 bis 411 der Prourokinase und
X eine der üblichen L-α-Aminosäuren
darstellen, wobei jedoch X nicht Arg sein darf, wenn Y Wasserstoff ist.

2. DNA-Sequenzen, die für die Polypeptide gemäß Anspruch 1 kodieren.

3. Vektoren, die Gensequenzen enthalten, die für die Polypeptide gemäß Anspruch 1 kodieren.

4. Gentechnologisches Verfahren zur Herstellung von Peptidsequenzen nach Anspruch 1, dadurch gekennzeichnet, daß man in einem Wirtsorganismus ein Gen zur Expression bringt, das für die Peptidsequenzen nach Anspruch 1 kodiert.

5. Arzneimittel enthaltend mindestens ein Polypeptid gemäß Anspruch 1, gegebenenfalls in einem pharmazeutisch verträglichen Träger oder Bindemittel.

6. Arzneimittel gemäß Anspruch 5, enthaltend die Kombination aus mindestens einem Polypeptid gemäß Anspruch 1 und einem anderen Fibrinolytikum.

870510

BASF Aktiengesellschaft                          O.Z. 0050/39530

Abb. 1: Aminosäuresequenz der reifen Prourokinase

                                              10
    Ser-Asn-Glu-Leu-His-Gln-Val-Pro-Ser-Asn-
                                              20
    Cys-Asp-Cys-Leu-Asn-Gly-Gly-Thr-Cys-Val-
                                              30
    Ser-Asn-Lys-Tyr-Phe-Ser-Asn-Ile-His-Trp-
                                              40
    Cys-Asn-Cys-Pro-Lys-Lys-Phe-Gly-Gly-Gln-
                                              50
    His-Cys-Glu-Ile-Asp-Lys-Ser-Lys-Thr-Cys-
                                              60
    Tyr-Glu-Gly-Asn-Gly-His-Phe-Tyr-Arg-Gly-
                                              70
    Lys-Ala-Ser-Thr-Asp-Thr-Met-Gly-Arg-Pro-
                                              80
    Cys-Leu-Pro-Trp-Asn-Ser-Ala-Thr-Val-Leu-
                                              90
    Gln-Gln-Thr-Tyr-His-Ala-His-Arg-Ser-Asp-
                                              100
    Ala-Leu-Gln-Leu-Gly-Leu-Gly-Lys-His-Asn-
                                              110
    Tyr-Cys-Arg-Asn-Pro-Asp-Asn-Arg-Arg-Arg-
                                              120
    Pro-Trp-Cys-Tyr-Val-Gln-Val-Gly-Leu-Lys-
                                              130
    Pro-Leu-Val-Gln-Glu-Cys-Met-Val-His-Asp-
                                              140
    Cys-Ala-Asp-Gly-Lys-Lys-Pro-Ser-Ser-Pro-
                                              150
    Pro-Glu-Glu-Leu-Lys-Phe-Gln-Cys-Gly-Gln-
                                              160
    Lys-Thr-Leu-Arg-Pro-Arg-Phe-Lys-Ile-Ile-
                                              170
    Gly-Gly-Glu-Phe-Thr-Thr-Ile-Glu-Asn-Gln-
                                              180
    Pro-Trp-Phe-Ala-Ala-Ile-Tyr-Arg-Arg-His-
                                              190
    Arg-Gly-Gly-Ser-Val-Thr-Tyr-Val-Cys-Gly-
                                              200
    Gly-Ser-Leu-Ile-Ser-Pro-Cys-Trp-Val-Ile-
                                              210
    Ser-Ala-Thr-His-Cys-Phe-Ile-Asp-Tyr-Pro-

BASF Aktiengesellschaft

870510

O.Z. 0050/39530

Abb. 1 (Fortsetzung)

220
Lys-Lys-Glu-Asp-Tyr-Ile-Val-Tyr-Leu-Gly-
230
Arg-Ser-Arg-Leu-Asn-Ser-Asn-Thr-Gln-Gly-
240
Glu-Met-Lys-Phe-Glu-Val-Glu-Asn-Leu-Ile-
250
Leu-His-Lys-Asp-Tyr-Ser-Ala-Asp-Thr-Leu-
260
Ala-His-His-Asn-Asp-Ile-Ala-Leu-Leu-Lys-
270
Ile-Arg-Ser-Lys-Glu-Gly-Arg-Cys-Ala-Gln-
280
Pro-Ser-Arg-Thr-Ile-Gln-Thr-Ile-Cys-Leu-
290
Pro-Ser-Met-Tyr-Asn-Asp-Pro-Gln-Phe-Gly-
300
Thr-Ser-Cys-Glu-Ile-Thr-Gly-Phe-Gly-Lys-
310
Glu-Asn-Ser-Thr-Asp-Tyr-Leu-Tyr-Pro-Glu-
320
Gln-Leu-Lys-Met-Thr-Val-Val-Lys-Leu-Ile-
330
Ser-His-Arg-Glu-Cys-Gln-Gln-Pro-His-Tyr-
340
Tyr-Gly-Ser-Glu-Val-Thr-Thr-Lys-Met-Leu-
350
Cys-Ala-Ala-Asp-Pro-Gln-Trp-Lys-Thr-Asp-
360
Ser-Cys-Gln-Gly-Asp-Ser-Gly-Gly-Pro-Leu-
370
Val-Cys-Ser-Leu-Gln-Gly-Arg-Met-Thr-Leu-
380
Thr-Gly-Ile-Val-Ser-Trp-Gly-Arg-Gly-Cys-
390
Ala-Leu-Lys-Asp-Lys-Pro-Gly-Val-Tyr-Thr-
400
Arg-Val-Ser-His-Phe-Leu-Pro-Trp-Ile-Arg-
410
Ser-His-Thr-Lys-Glu-Glu-Asn-Gly-Leu-Ala-
411
Leu

Abb. 2                                         O.Z. 0050/39530

```
    gtccccgcagcgccgtcgcgccctcctgccgcaggccaccgaggccgccgccgtctagcg
1   ---------+---------+---------+---------+---------+---------+ 60
    caggggcgtcgcggcagcgcgggaggacggcgtccggtggctccggcggcggcagatcgc


    ccccgacctcgccaccatgagagccctgctggcgcgcctgcttctctgcgtcctggtcgt
61  ---------+---------+---------+---------+---------+---------+ 120
    ggggctggagcggtggtactctcgggacgaccgcgcggacgaagagacgcaggaccagca
```

                                  T
                                  a
                                  q
                                  I

```
    gagcgactccaaaggcagcaatgaacttcatcaagttccatcgaactgtgactgtctaaa
121 ---------+---------+---------+---------+---------+---------+ 180
    ctcgctgaggtttccgtcgttacttgaagtagttcaaggtagcttgacactgacagattt

         SerAsnGluLeuHisGlnValProSerAsnCysAspCysLeuAsn -

    tggaggaacatgtgtgtccaacaagtacttctccaacattcactggtgcaactgcccaaa
181 ---------+---------+---------+---------+---------+---------+ 240
    acctccttgtacacacaggttgttcatgaagaggttgtaagtgaccacgttgacgggttt

     GlyGlyThrCysValSerAsnLysTyrPheSerAsnIleHisTrpCysAsnCysProLys -

    gaaattcggagggcagcactgtgaaatagataagtcaaaaacctgctatgaggggaatgg
241 ---------+---------+---------+---------+---------+---------+ 300
    ctttaagcctcccgtcgtgacactttatctattcagttttttggacgatactcccttacc

     LysPheGlyGlyGlnHisCysGluIleAspLysSerLysThrCysTyrGluGlyAsnGly -

    tcacttttaccgaggaaaggccagcactgacaccatgggccggccctgcctgccctggaa
301 ---------+---------+---------+---------+---------+---------+ 360
    agtgaaaatggctcctttccggtcgtgactgtggtacccggccgggacggacgggacctt

     HisPheTyrArgGlyLysAlaSerThrAspThrMetGlyArgProCysLeuProTrpAsn -

    ctctgccaatgtccttcagcaaacgtaccatgcccacagatctgatgctcttcagctggg
361 ---------+---------+---------+---------+---------+---------+ 420
    gagacggttacaggaagtcgtttgcatggtacgggtgtctagactacgagaagtcgaccc

     SerAlaAsnValLeuGlnGlnThrTyrHisAlaHisArgSerAspAlaLeuGlnLeuGly -

    cctggggaaacataattactgcaggaacccagacaaccggaggcgaccctggtgctatgt
421 ---------+---------+---------+---------+---------+---------+ 480
    ggacccctttgtattaatgacgtccttgggtctgttggcctccgctgggaccacgataca

     LeuGlyLysHisAsnTyrCysArgAsnProAspAsnArgArgArgProTrpCysTyrVal -
```

Abb. 2 Forts.

```
       gcaggtgggcctaaagccgcttgtccaagagtgcatggtgcatgactgcgcagatggaaa
   481 ---------+---------+---------+---------+---------+---------+ 540
       cgtccacccggatttcggcgaacaggttctcacgtaccacgtactgacgcgtctacctttt

       GlnValGlyLeuLysProLeuValGlnGluCysMetValHisAspCysAlaAspGlyLys -

                                         B
                                         a
                                         l
                                         I
       aaagccctcctctcctccagaagaattaaaatttcagtgtggccaaaagactctgaggcc
   541 ---------+---------+---------+---------+---------+---------+ 600
       tttcgggaggagaggaggtcttcttaattttaaagtcacaccggttttctgagactccgg

       LysProSerSerProProGluGluLeuLysPheGlnCysGlyGlnLysThrLeuArgPro -

                       E                     T
                       c                     a
                       o                     q
                       R                     I
                       I
       ccgctttaagattattggggagaattcaccaccatcgagaaccagccctggtttgcggc
   601 ---------+---------+---------+---------+---------+---------+ 660
       ggcgaaattctaataaccccctcttaagtggtggtagctcttggtcgggaccaaacgccg

       ArgPheLysIleIleGlyGlyGluPheThrThrIleGluAsnGlnProTrpPheAlaAla -

       catctacaggaggcaccgggggggctctgtcacctacgtgtgtggaggcagcctcatcag
   661 ---------+---------+---------+---------+---------+---------+ 720
       gtagatgtcctccgtggcccccccgagacagtggatgcacacacctccgtcggagtagtc

       IleTyrArgArgHisArgGlyGlySerValThrTyrValCysGlyGlySerLeuIleSer -

       cccttgctgggtgatcagcgccacacactgcttcattgattacccaaagaaggaggacta
   721 ---------+---------+---------+---------+---------+---------+ 780
       gggaacgacccactagtcgcggtgtgtgacgaagtaactaatgggtttcttcctcctgat

       ProCysTrpValIleSerAlaThrHisCysPheIleAspTyrProLysLysGluAspTyr -

       catcgtctacctgggtcgctcaaggcttaactccaacacgcaaggggagatgaagtttga
   781 ---------+---------+---------+---------+---------+---------+ 840
       gtagcagatggacccagcgagttccgaattgaggttgtgcgttcccctctacttcaaact

       IleValTyrLeuGlyArgSerArgLeuAsnSerAsnThrGlnGlyGluMetLysPheGlu -

       ggtggaaaacctcatcctacacaaggactacagcgctgacacgcttgctcaccacaacga
   841 ---------+---------+---------+---------+---------+---------+ 900
       ccaccttttggagtaggatgtgttcctgatgtcgcgactgtgcgaacgagtggtgttgct

       ValGluAsnLeuIleLeuHisLysAspTyrSerAlaAspThrLeuAlaHisHisAsnAsp -

       cattgccttgctgaagatccgttccaaggagggcaggtgtgcgcagccatcccggactat
   901 ---------+---------+---------+---------+---------+---------+ 960
       gtaacggaacgacttctaggcaaggttcctcccgtccacacgcgtcggtagggcctgata

       IleAlaLeuLeuLysIleArgSerLysGluGlyArgCysAlaGlnProSerArgThrIle -
```

Abb. 2 Forts.

```
                              T
                              a
                              q
                              I
        acagaccatctgcctgccctcgatgtataacgatccccagtttggcacaagctgtgagat
   961  ---------+---------+---------+---------+---------+---------+  1020
        tgtctggtagacggacgggagctacatattgctaggggtcaaaccgtgttcgacactcta

        GlnThrIleCysLeuProSerMetTyrAsnAspProGlnPheGlyThrSerCysGluIle -

                              E
                              c
                              o
                              R
                              I
        cactggctttggaaaagagaattctaccgactatctctatccggagcagctgaaaatgac
  1021  ---------+---------+---------+---------+---------+---------+  1080
        gtgaccgaaacctttctcttaagatggctgatagagataggcctcgtcgacttttactg

        ThrGlyPheGlyLysGluAsnSerThrAspTyrLeuTyrProGluGlnLeuLysMetThr -

        tgttgtgaagctgatttcccaccgggagtgtcagcagccccactactacggctctgaagt
  1081  ---------+---------+---------+---------+---------+---------+  1140
        acaacacttcgactaaagggtggccctcacagtcgtcggggtgatgatgccgagacttca

        ValValLysLeuIleSerHisArgGluCysGlnGlnProHisTyrTyrGlySerGluVal -

        caccaccaaaatgctgtgtgctgctgacccacagtggaaaacagattcctgccagggaga
  1141  ---------+---------+---------+---------+---------+---------+  1200
        gtggtggttttacgacacacgacgactgggtgtcaccttttgtctaaggacggtccctct

        ThrThrLysMetLeuCysAlaAlaAspProGlnTrpLysThrAspSerCysGlnGlyAsp -

        ctcagggggacccctcgtctgttccctccaaggccgcatgactttgactggaattgtgag
  1201  ---------+---------+---------+---------+---------+---------+  1260
        gagtccccctggggagcagacaagggaggttccggcgtactgaaactgaccttaacactc

        SerGlyGlyProLeuValCysSerLeuGlnGlyArgMetThrLeuThrGlyIleValSer -

        ctggggccgtggatgtgccctgaaggacaagccaggcgtctacacgagagtctcacactt
  1261  ---------+---------+---------+---------+---------+---------+  1320
        gaccccggcacctacacgggacttcctgttcggtccgcagatgtgctctcagagtgtgaa

        TrpGlyArgGlyCysAlaLeuLysAspLysProGlyValTyrThrArgValSerHisPhe -

                              B
                              a
                              m
                              H
                              I
        cttaccctggatccgcagtcacaccaaggaagagaatggcctggccctctgagggtcccc
  1321  ---------+---------+---------+---------+---------+---------+  1380
        gaatgggacctaggcgtcagtgtggttccttctcttaccggaccgggagactcccagggg

        LeuProTrpIleArgSerHisThrLysGluGluAsnGlyLeuAlaLeuEnd
```

Abb. 2 Forts.                                          O.Z. 0050/39530

```
      agggaggaaacgggcaccacccgctttcttgctggttgtcattttgcagtagagtcatc
1381  ---------+---------+---------+---------+---------+---------+ 1440
      tccctcctttgcccgtggtgggcgaaagaacgaccaacagtaaaaacgtcatctcagtag


      tccatcagctgtaagaagagactgggaagataggctctgcacagatggatttgcctgtgc
1441  ---------+---------+---------+---------+---------+---------+ 1500
      aggtagtcgacattcttctctgacccttctatccgagacgtgtctacctaaacggacacg
```

FIG.3

O.Z. 0050/39530

FIG.4

FIG.5

# FIG. 6

1. Bal I
2. EcoR1 partiell
   großes Fragment

+ oligo  84/85  (Y=Lys$^{156}$) oder
  oligo  86/87  (Y=Gln$^{156}$) oder
  oligo  88/89  (Y=Leu$^{156}$)

Ligation

# FIG.7

Inkubationszeit (min.)